# EUROPEAN PATENT SPECIFICATION

(11) **EP 3 568 098 B1**
(45) Date of publication and mention of the grant of the patent: **30.03.2022**
(21) Application number: 19726879.0
(22) Date of filing: 21.03.2019
(51) Int. Cl.: A61B 18/00, A61B 18/14, A61B 18/02, A61B 18/04, A61B 18/08, A61B 90/00, A61B 6/12, A61B 6/00, A61B 18/12, A61B 34/20

(54) **IRRIGATED ELECTROPHYSIOLOGY CATHETER WITH DISTINGUISHABLE ELECTRODES FOR MULTI-ELECTRODE IDENTIFICATION AND ORIENTATION UNDER 2-D VISUALIZATION**
BEWÄSSERTER ELEKTROPHYSIOLOGISCHER KATHETER MIT UNTERSCHEIDBAREN ELEKTRODEN ZUR MULTIELEKTRODENIDENTIFIKATION UND -AUSRICHTUNG UNTER 2D-VISUALISIERUNG
CATHÉTER D'ÉLECTROPHYSIOLOGIE IRRIGUÉ COMPORTANT DES ÉLECTRODES IDENTIFIABLES POUR UNE IDENTIFICATION ET UNE ORIENTATION MULTI-ÉLECTRODES EN VISUALISATION 2D

(30) Priority: 28.03.2018 US 201815939154
(43) Date of publication of application: 20.11.2019
(73) Proprietor: Biosense Webster (Israel) Ltd., 2066717 Yokneam (IL)
(72) Inventor: CLARK, Jeffrey L., Irwindale, Clifornia 91706 (US)
(74) Representative: Carpmaels & Ransford LLP
(86) International application number: PCT/IB2019/052313
(87) International publication number: WO 2019/186336

(56) References cited:
- WO-A1-2015/200518
- WO-A1-2015/200518
- US-A1- 2014 288 546
- US-A1- 2014 288 546
- US-A1- 2017 042 614
- US-A1- 2017 042 614

## Description

### FIELD

The subject matter disclosed herein relates to electrophysiologic catheters, particularly those capable of ablating cardiac tissue.

### BACKGROUND

Ablation of cardiac tissue has been used to treat cardiac arrhythmias. Ablative energies are typically provided to cardiac tissue by a tip portion which can deliver ablative energy alongside the tissue to be ablated. Some of these catheters administer ablative energy from various electrodes three-dimensional structures. Ablative procedures incorporating such catheters may be visualized using fluoroscopy.

US 2017/042614 A1 describes cardiac tissue ablation catheters including an inflatable and flexible toroidal or spherically shaped balloon disposed at a distal region of an elongate member, a flexible circuit carried by an outer surface of the balloon, the flexible circuit including, a plurality of flexible branches conforming to the radially outer surface of the balloon, each of the plurality of flexible branches including a substrate, a conductive trace carried by the substrate, and an ablation electrode carried by the substrate.

WO 2015/200518 A1 describes tissue ablation devices, systems, and methods for monitoring or analyzing one or more aspects of tissue ablation.

### SUMMARY OF THE DISCLOSURE

While fluoroscopic visualization of cardiac devices is an established means of visualization that is simpler to use than a magnetic location and impedance system for electrophysiology procedures (EP), it is only currently used to judge overall device location relative to the cardiac silhouette. When contemplating the use of multi-electrode devices for EP with fluoroscopic imaging, the limitations of fluoroscopic visualization became apparent. This is because the fluoroscopic image is strictly 2D and lacks any visual cues relating to depth. And in the world of human visual perception, this is known as a bi-stable image. To correctly resolve the relative orientation of the device in a bi-stable image, the human brain needs queues to orient the image. In devising an electrophysiology catheter with multiple electrodes disposed in an array about the longitudinal axis of the balloon for use in ablation of the PV, applicant has discovered that with a bi-stable image, there is a need to determine the orientation of the electrode and as well as which electrode should be energized. That is, depending on the electroanatomical mapping of the subject, it may not be necessary to energize all of the electrodes array which typically would form a circular ablated tissue under bi-stable visualization. Additionally, upon determining that certain electrodes may contact tissue whereas others may not, it may be desirable to activate only those electrodes in contact with the tissue. As such, applicant has configured the catheter to allow the operator to determine which electrode in the electrode array of the balloon would need to be energized under bi-stable visualization so as to minimize the application of excessive energy to tissues that do not need to be ablated.

Accordingly, applicant has devised an electrophysiology catheter having a balloon with a membrane according to the present invention. The balloon has a distal end and a proximal end defining a longitudinal axis. A first substrate is disposed on the membrane. The first substrate includes a first radiopaque marker of a first form disposed thereon. A second substrate is also disposed upon the membrane. The second substrate includes a second radiopaque marker of a second form disposed thereon. The second form is different from the first form. The first form includes a first polygon and the second form includes a second polygon, wherein the second polygon is asymmetric about a centerline that is parallel to the longitudinal axis of the balloon, wherein the second polygon includes an arrowhead, and wherein the second polygon points toward a third substrate. The third substrate includes a third radiopaque marker of a third form disposed thereon, the third form being different from the first form and the second form, and wherein the third form includes a third polygon. The third polygon is asymmetric about a centerline that is parallel to the longitudinal axis of the balloon, wherein the third polygon includes an arrowhead, and wherein the third polygon points toward an ultimate substrate.

In some examples, the first form may be a first numeral and the second form may be a second numeral. For example, the first numeral may be a "1" and the second numeral may be a numeral greater than "1," e.g., a "4." These distinct forms allow for the operator to distinguish which electrode is spaced relative to the other electrode as arrayed on the balloon, as well as allowing a technique to recognize all other electrodes arrayed on the balloon. In one embodiment, two markers with distinct forms on two respective electrodes are separated by at least an electrode without any distinct marker form. Alternatively, two markers with distinct forms on two respective electrodes are separated by one or more electrodes with a marker of another form. In some embodiments, two markers with distinct forms on two respective electrodes are separated by two electrodes without any distinct form. Alternatively, two markers with distinct forms on two respective electrodes are separated by two electrodes with a marker of another form utilized for the other electrodes. As described herein, the markers can be disposed on the substrate as a distinct member from the electrode or formed as part of the electrode.

The first polygon may be rectangular or it may be a rectangle, and the second polygon may be triangular or it may be a triangle. These polygons may be solid or filled, or they may be hollow or unfilled. Thus, the first polygon may be solid, and the second polygon may be hollow, or it may instead be solid.

In those examples where the first electrode and second electrode include markers in the form of alphanumeric symbols, the third electrode may also include a marker in the form of an alphanumeric symbol. For example, the form of the third marker may be a numeral that is greater than the numeral of the second marker. Thus, the third marker may have the form of, e.g., a "7." The third marker may be of a triangular form, e.g., it may be a triangle. As with the first polygonal marker and second polygonal marker, the third polygonal marker may be solid or hollow. In certain embodiments, the first marker is solid and the second and third markers are both hollow. In other embodiments, the first marker is hollow and the second and third markers are both solid. In other embodiments, the first marker and the second marker are both solid and the third marker is hollow.

Each of the substrates may be disposed on the membrane of the catheter's balloon in a circumferential pattern. Thus, in certain examples, the first electrode, second electrode, third electrode, and ultimate electrode are disposed circumferentially about the membrane such that the ultimate electrode is disposed on the membrane between the third electrode and the first electrode. A fourth electrode may be disposed on the membrane between the first electrode and the second electrode, and a fifth electrode may be disposed on the membrane between the second and third electrode. The fourth electrode may include a radiopaque marker of a fourth form, the fifth electrode may include a radiopaque marker of a fifth form, and the ultimate electrode may include a radiopaque marker of an ultimate form.

The first form may additionally include a first line, the second form may additionally include a second line, and the third form may additionally include a third line.

A sixth electrode may be disposed on the membrane between the first electrode and the fourth electrodes, a seventh electrode may be disposed on the membrane between the second and fifth electrodes, an eighth electrode may be disposed on the membrane between the third and ultimate electrodes, and a ninth electrode may be disposed on the membrane between the eighth and ultimate electrodes.

The first line may be a solid line, the second line may be a solid line, the third line may be a solid line, the fourth line may be a dotted line, the fifth line may be a dotted line, and the ultimate line may be a dotted line. The fourth line may be a solid line, the second line may be a dotted line, the fifth line may be a dotted line, the third line may be a hashed line, and the ultimate line may be a hashed line.

For radiopaque markers having forms including lines, the lengths of the lines may be the same or they may vary. For example, the fourth line may be longer than the first line, the second line may be longer than the fourth line, the fifth line may be longer than the second line, the third line may be longer than the fifth line, and the ultimate line may be longer than the third line.

For polygonal markers, the first polygon may be disposed proximate to an end of the first line, such as the proximal end of the first line. The second polygon may be disposed proximate to an end of the second line, such as the proximal end of the second line. Alternatively, second polygon may be disposed proximate to a midpoint of the second line.

For polygonal markers, the first polygon may be disposed proximate to a midpoint of the first line and second polygon may be disposed proximate to an end of the second line, such as the proximate end of the second line. Alternatively, the second polygon may be disposed proximate to a midpoint of the second line.

The catheter may be part of an electrophysiology system. In addition to the catheter, the electrophysiology system may comprise an ablation module. The ablation module may include a radio-frequency signal generator, a first output, and a second output. The system may also include a first wire in an electrical path between the first output and a first electrode disposed on the balloon. The system may also comprise a second wire in an electrical path between the second output and a second electrode disposed on the balloon. In some embodiments, the first wire may be at least partially disposed within the catheter and the second wire may be at least partially disposed within the catheter. Further, the first electrode may include thereon a first radiopaque marker of a first form and the second electrode may include thereon a second radiopaque marker of a second form that is different than the first form. For example, the first form may be a rectangle and the second form may be a triangle. In some embodiments the rectangle is solid. In some embodiments the triangle is also solid. However, in other embodiments the triangle may be hollow.

The catheter may be employed in a method and variations thereof for ablating tissue. In certain variations, the catheter may be provided. A balloon of the catheter may include disposed thereon a first electrode and a second electrode. The first electrode may be connected to a radio-frequency signal generator via a first switch. The second electrode may be connected to the same or a different radio-frequency generator via a second switch. The first electrode may include a first radiopaque marker at least partially disposed thereon. The second electrode may include a second radiopaque marker at least partially disposed thereon. The balloon may be positioned proximate to a tissue within a subject's anatomy, e.g., proximate to or within a pulmonary vein ostium in the subject's heart. The balloon may be visualized therein using a medical-visualization technique, e.g., MRI or fluoroscopy. From this visualization, a user may determine that the first marker contacts the tissue, and then activate the first electrode by closing the first switch. In some variations, the user may also determine that the second marker does not contact tissue, such that it may be undesirable to activate the second electrode. However, in other variations, the user may determine that the second marker contacts the tissue and then activate the second electrode by closing the second switch.

Because fluoroscopic images may be bi-stable a user may have difficulty determining that the first switch may be used to activate the first electrode and that the second switch may be used to activate the second electrode. Using the radiopaque markers, however, the user may determine that the first switch activates the first electrode and that the second switch activates the second electrode. For example, the user may know that the first marker includes a first form that corresponds to the first switch such that the user may determine which switch to activate based on the correlating the first form to the first switch. In some variations, the user may also determine that the second switch activates the second electrode by applying a right-hand rule based in part on knowing that the first form correlates to the first switch.

Further, in those embodiments that include a single radio-frequency generator, each electrode may receive radio-frequency energy having a similar frequency. However, in those embodiments that include more than one radio-frequency generator, e.g., one generator per electrode, each electrode may receive radio-frequency energy having different frequencies.

In yet a further aspect, we have devised method for ablating tissue that can be achieved by: positioning an expandable member proximate to a tissue within a subject's anatomy, the expandable member having a longitudinal axis and including a plurality of electrodes disposed about the longitudinal axis, each electrode capable of being energized independently, at least a first electrode electrically connected to a first switch, the first electrode having a first radiopaque marker and a second electrode electrically connected to a second switch, the second electrode having a second radiopaque marker different from the first radiopaque marker; viewing a fluoroscopic image of the expandable member; determining that the first marker contacts the tissue; and activating the first electrode with the first switch.

Additionally, we have devised a method of applying energy to tissue in a subject that can be achieved by: positioning an expandable member proximate to a tissue within a subject's anatomy, the expandable member having a longitudinal axis and including a plurality of electrodes disposed about the longitudinal axis, each electrode capable of being energized independently, at least a first electrode electrically connected to a first switch, the first electrode having a first radiopaque marker and a second electrode electrically connected to a second switch, the second electrode having a second radiopaque marker different from the first radiopaque marker; viewing an image of the expandable member; determining that one or more electrodes adjacent one of the first marker or the second marker contacts the tissue; and energizing the one or more electrodes to ablate the tissue.

Finally, we have devised a method of applying energy to targeted tissue region without damaging adjacent anatomical structures in a subject by: positioning an expandable member proximate to the left atrium, the expandable member having a longitudinal axis and including a plurality of electrodes disposed about the longitudinal axis, each electrode capable of being energized independently, the plurality of electrodes including a first electrode having a first radiopaque marker and a second electrode having a second radiopaque marker different from the first radiopaque marker; viewing an image of the expandable member as well as the first and second radiopaque markers in the left atrium; determining an orientation of the first and second radiopaque markers with respect to a portion of the left atrium closest to the esophagus, phrenic nerve, or lung, of the subject; moving one of the first and second radiopaque marker to a portion of the left atrium closest to the esophagus, phrenic nerve or lung; and energizing one or more electrodes indexed to the one of the radiopaque markers proximate the portion close to the esophagus, phrenic nerve, or lung, at a lower energization setting as compared to other electrodes to create a transmural lesion in the left atrium with little or no effect to adjacent anatomical structures such as, for example, the esophagus, phrenic nerve or lung.

### BRIEF DESCRIPTION OF THE DRAWINGS

While the specification concludes with claims, which particularly point out and distinctly claim the subject matter described herein, it is believed the subject matter will be better understood from the following description of certain examples taken in conjunction with the accompanying drawings, in which like reference numerals identify the same elements and in which:
FIG. 1 is a schematic illustration of an invasive medical procedure;
FIG. 2 is a top view of a catheter with a balloon in an expanded state, in use with a lasso catheter;
FIG. 3 is a perspective view of a balloon of FIG. 2, along with the lasso catheter;
FIG. 4 is a side view of a distal end of the catheter of FIG. 2 deployed in the region of a pulmonary vein and its ostium;
FIG. 5 is a top plan view of a plurality of flex circuit electrode assemblies prior to being assembled onto the balloon of FIG. 3;
FIG. 5A is a top plan view of a plurality of flex circuit electrode assemblies that includes radiopaque markers prior to being assembled onto the balloon of FIG. 3;
FIG. 5B is a representation of a fluoroscopic image of the balloon of FIG. 3 that includes the flex circuit electrode assemblies of FIG. 5A.
FIG. 6 is a perspective detail view of a flex circuit electrode assembly after being assembled on the balloon of FIG. 3;
FIG. 7A is a magnified view of a portion of one of the flex circuit electrode assemblies of FIG. 5 that includes a radiopaque marker of a form;
FIG. 7B is a magnified view of a portion of one of the flex circuit electrode assemblies of FIG. 5 that includes a radiopaque marker of another form;
FIG. 7C is a magnified view of a portion of one of the flex circuit electrode assemblies of FIG. 5 that includes a radiopaque marker of another form;
FIG. 7D is a magnified view of a portion of one of the flex circuit electrode assemblies of FIG. 5 that includes a radiopaque marker of another form;
FIG. 7E is a magnified view of a portion of one of the flex circuit electrode assemblies of FIG. 5 that includes a radiopaque marker of another form;
FIG. 7F is a magnified view of a portion of one of the flex circuit electrode assemblies of FIG. 5 that includes a radiopaque marker of another form;
FIG. 7G is a magnified view of a portion of one of the flex circuit electrode assemblies of FIG. 5 that includes a radiopaque marker of another form;
FIG. 7H is a magnified view of a portion of one of the flex circuit electrode assemblies of FIG. 5 that includes a radiopaque marker of another form;
FIG. 7I is a magnified view of a portion of one of the flex circuit electrode assemblies of FIG. 5 that includes a radiopaque marker of another form;
FIG. 7J is a magnified view of a portion of one of the flex circuit electrode assemblies of FIG. 5 that includes a radiopaque marker of another form;
FIG. 7K is a magnified view of a portion of one of the flex circuit electrode assemblies of FIG. 5 that includes a radiopaque marker of another form;
FIG. 8A shows the balloon of FIG. 3 as including radiopaque markers according to one embodiment;
FIG. 8B is an alternative rotated view of the balloon of FIG. 8A;
FIG. 9A shows the balloon of FIG. 3 as including radiopaque markers according to another embodiment;
FIG. 9B is an alternative rotated view of the balloon of FIG. 9A; and
FIG. 10 shows the balloon of FIG. 3 as including radiopaque markers according to a different embodiment.
FIGS. 11, 12A, 12B, 13 and 14 illustrate yet more embodiments of the radiopaque markers that can be utilized with the balloon.
FIG. 15 illustrates a sectional representation of the balloon of Fig. 5B in a subject's left atrium as well as other anatomical structures adjacent the heart.

### MODES OF CARRYING OUT THE INVENTION

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±10% of the recited value, e.g. "about 90%" may refer to the range of values from 81% to 99%. In addition, as used herein, the terms "patient," "host," "user," and "subject" refer to any human or animal subject and are not intended to limit the systems or methods to human use, although use of the subject invention in a human patient represents a preferred embodiment.

### Overview

Ablation of cardiac tissue to correct a malfunctioning heart is a well-known procedure for implementing such a correction. Typically, to successfully ablate, cardiac electropotentials need to be measured at various locations of the myocardium. In addition, temperature measurements during ablation provide data enabling the efficacy of the ablation to be measured. Typically, for an ablation procedure, the electropotentials and the temperatures are measured before, during, and after the actual ablation.

In contrast with prior art systems that use two or more separate instructions (e.g., one for the electropotentials and temperature measurements, and another for the ablation), embodiments disclosed herein facilitate the two measurements, and in addition enable ablation using radiofrequency electromagnetic energy, using a single catheter. The catheter has a lumen, and a balloon is deployed through the catheter lumen (the balloon travels through the lumen in a collapsed, uninflated configuration, and the balloon is inflated on exiting the lumen). The balloon has an exterior wall or membrane and has a distal end and a proximal end which define a longitudinal axis that extends the lumen.

A multi-layer flexible metal structure is attached to an exterior wall or membrane of the balloon. The structure comprises a plurality of electrode groups arranged circumferentially about the longitudinal axis, where each electrode group comprises multiple ablation electrodes, typically arranged longitudinally.

Each electrode group may also include at least one micro-electrode that is insulated physically and electrically from the ablation electrodes in its group.

Each electrode group may also include at least a thermocouple.

In some embodiments, each electrode group has a micro-electrode and a thermocouple formed at a common location.

Using a single catheter, with the three functionalities of ability to perform ablation, electropotential measurement, and temperature measurement, simplifies cardiac ablation procedures.

### System Description

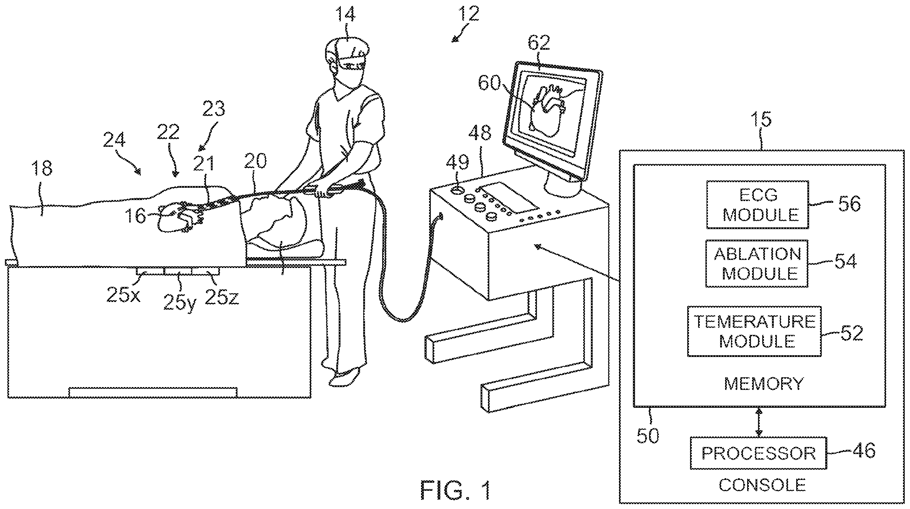

FIG. 1 is a schematic illustration of an invasive medical procedure using apparatus 12, according to an embodiment. The procedure is performed by a medical professional 14, and, by way of example, the procedure in the description hereinbelow is assumed to comprise ablation of a portion of a myocardium 16 of the heart of a human patient 18. However, it is understood that embodiments disclosed herein are not merely applicable to this specific procedure, and may include substantially any procedure on biological tissue or on non-biological materials.

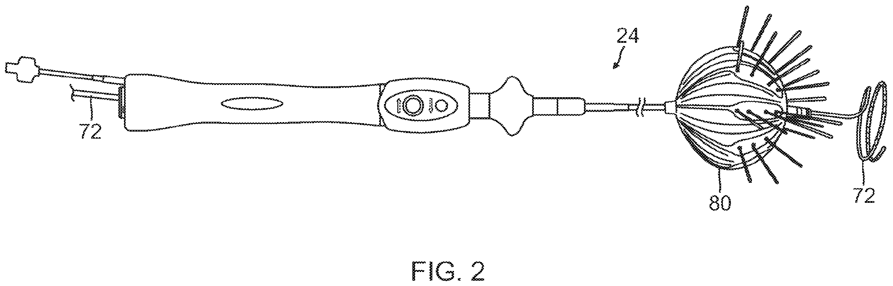

To perform the ablation, medical professional 14 inserts a probe 20 into a sheath 21 that has been pre-positioned in a lumen of the patient. Sheath 21 is positioned so that a distal end 22 of probe 20 enters the heart of the patient. A diagnostic/therapeutic catheter 24 (e.g., a balloon catheter), which is described in more detail below with reference to FIG. 2, is deployed through a lumen 23 of the probe 20, and exits from a distal end of the probe 20.

As shown in FIG. 1, apparatus 12 is controlled by a system processor 46, which is in an operating console 15 of the apparatus. Console 15 comprises controls 49 which are used by professional 14 to communicate with the processor. During the procedure, the processor 46 typically tracks a location and an orientation of the distal end 22 of the probe 20, using any method known in the art. For example, processor 46 may use a magnetic tracking method, wherein magnetic transmitters 25X, 25Y and 25Z external to the patient 18 generate signals in coils positioned in the distal end of the probe 20. The CARTO^{®} system (available from Biosense Webster, Inc. of Irvine, California) uses such a tracking method.

The software for the processor 46 may be downloaded to the processor in electronic form, over a network, for example. Alternatively, or additionally, the software may be provided on non-transitory tangible media, such as optical, magnetic, or electronic storage media. The tracking of the distal end 22 is may be displayed on a three-dimensional representation 60 of the heart of the patient 18 on a screen 62. However, it may be displayed two-dimensionally, e.g., by fluoroscopy or MRI

To operate apparatus 12, the processor 46 communicates with a memory 50, which has many modules used by the processor to operate the apparatus. Thus, the memory 50 comprises a temperature module 52, an ablation module 54, and an electrocardiograph (ECG) module 56, the functions of which are described below. The memory 50 typically comprises other modules, such as a force module for measuring the force on the distal end 22, a tracking module for operating the tracking method used by the processor 46, and an irrigation module allowing the processor to control irrigation provided for the distal end 22. For simplicity, such other modules are not illustrated in FIG. 1. The modules may comprise hardware as well as software elements. For example, module 54 may include a radio-frequency generator with at least one output or output channel, e.g., ten outputs or ten output channels. Each of the outputs may be separately and selectively activated or deactivated by a switch. That is, each switch may be disposed between the signal generator and a respective output. Thus, a generator with ten outputs would include ten switches. These outputs may each be individually coupled to electrodes on an ablation catheter, e.g., the ten electrodes 33 on balloon 80, described in further detail below. Such an electrical connection may be achieved by establishing an electrical path between each output and each electrode. For example, each output may be connected to a corresponding electrode by one or more wires or suitable electrical connectors. Thus, in some embodiments, an electrical path may include at least one wire. In some embodiments, the electrical path may further include an electrical connector and at least a second wire. Thus, electrodes 33 may be selectively activated and deactivated with the switches to receive radiofrequency energy separately from each of the other electrodes.

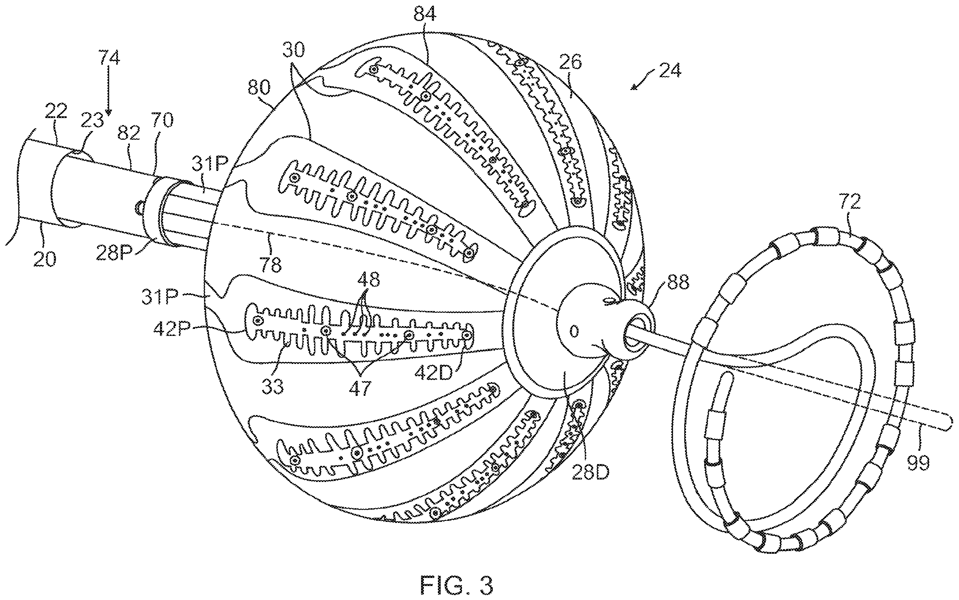

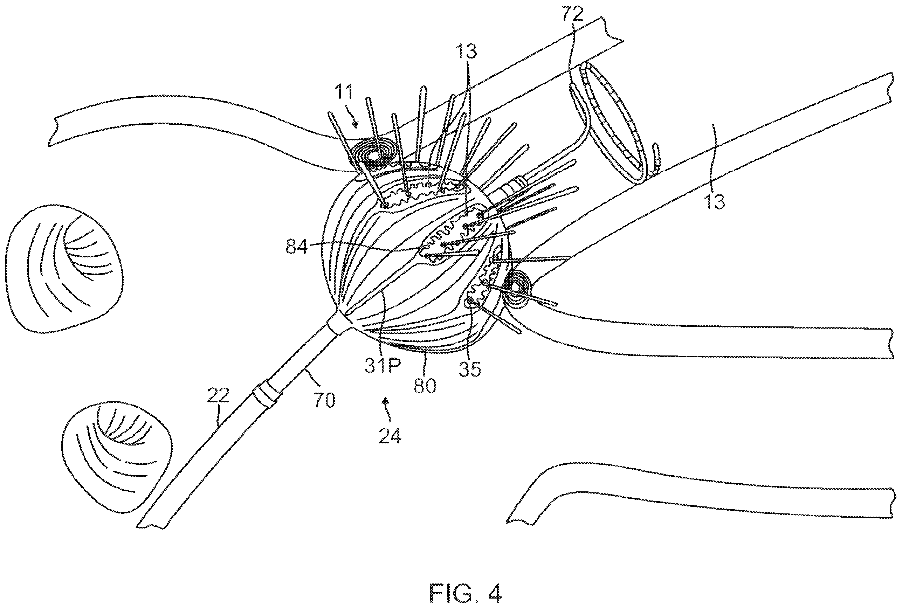

FIG. 3 is a schematic perspective view of the diagnostic/therapeutic catheter 24 in an expandable configuration in the form of a balloon in its expanded configuration, according to an embodiment. In a disclosed embodiment, where the diagnostic/therapeutic catheter 24 is used to ablate an ostium 11 of a lumen, such as a pulmonary vein 13, as shown in FIG. 4, the diagnostic/therapeutic catheter 24 is supported by a tubular shaft 70 having a proximal shaft portion 82 and a distal shaft end 88. The shaft 70 includes a hollow central tube 74, which permits a catheter to pass therethrough and past the distal shaft end 88. The catheter may be a focal linear catheter or a lasso catheter 72, as illustrated. The lasso catheter 72 may be inserted into the pulmonary vein to position the diagnostic/therapeutic catheter 24 correctly with respect to the ostium prior to ablation of the ostium. The distal lasso portion of the catheter 72 is typically formed of shape-memory retentive material such as nitinol. It is understood that the diagnostic/therapeutic catheter 24 may also be used with a linear or focal catheter 99 (as shown in broken lines in FIG. 3) in the PV or elsewhere in the heart. The focal catheter 99 may include a force sensor at its distal tip. Suitable force sending distal tips are disclosed in U.S. Patent No. 8,357,152, issued on January 22, 2013 to Govari et al., titled CATHETER WITH PRESSURE SENSING, and in U.S. Patent Application 2011/0130648, to Beeckler et al., filed Nov. 30, 2009, titled CATHETER WITH PRESSURE MEASURING TIP. Any catheter used in conjunction with the diagnostic/therapeutic catheter may have features and functions, including, for example, pressure sensing, ablation, diagnostic, e.g., navigation and pacing.

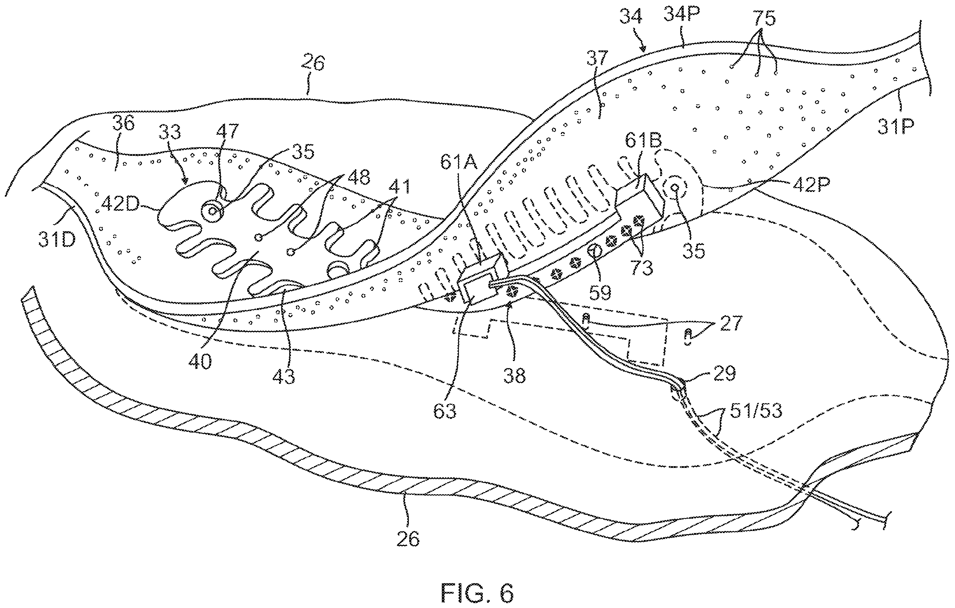

The balloon 80 of the diagnostic/therapeutic catheter 24 has an exterior wall or membrane 26 of a bio-compatible material, for example, formed from a plastic such as polyethylene terephthalate (PET), polyurethane or PEBAX^{®}. The shaft 70 and the distal shaft end 88 define a longitudinal axis 78 of the balloon 80. The balloon 80 is deployed, in a collapsed configuration, via the lumen 23 of the probe 20, and may be expanded after exiting from the distal end 22. The membrane 26 of the balloon 80 is formed with irrigation pores or apertures 27 (shown in FIG. 6) through which the fluid (e.g., saline) can exit from the interior of the balloon 80 to outside the balloon for cooling the tissue ablation site at the ostium. While FIG. 2 and FIG. 4 show fluid exiting the balloon 80 as jet streams, it is understood that the fluid may exit the balloon with any desired flow rate or pressure, including a rate where the fluid is seeping out of the balloon.

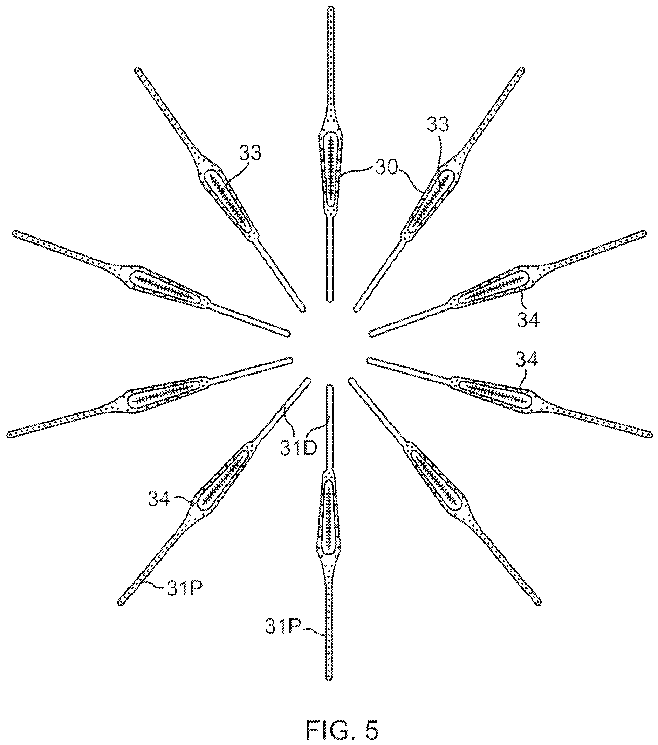

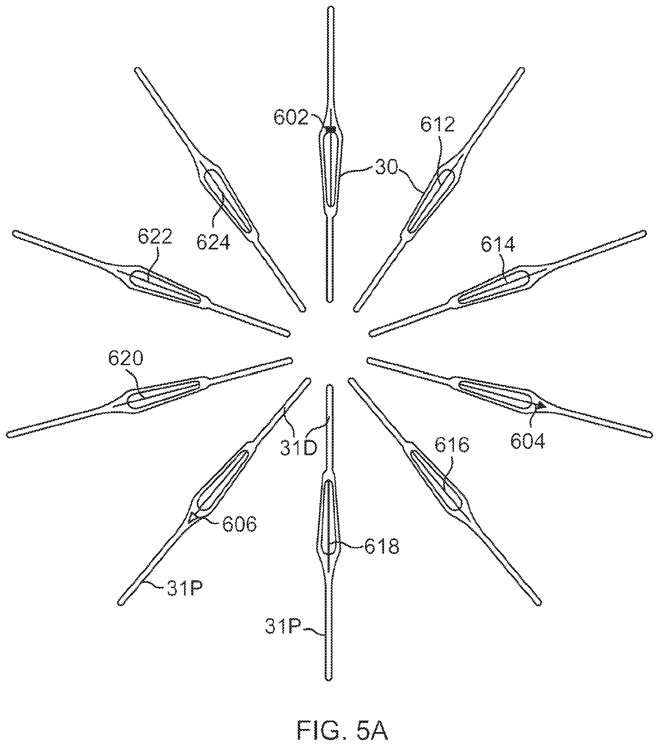

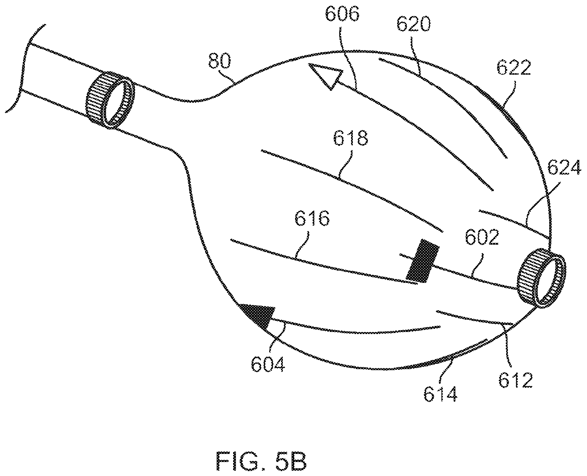

The membrane 26 supports and carries a combined electrode and temperature sensing member which is constructed as a multi-layer flexible circuit electrode assembly 84. The "flex circuit electrode assembly" 84 may have many different geometric configurations. In the illustrated embodiment, the flex circuit electrode assembly 84 has a plurality of radiating substrates or strips 30, as best seen in FIG. 5. The substrates 30 are evenly distributed about the distal end 88 and the balloon 80. Each substrate has wider proximal portion that gradually tapers to a narrower distal portion. FIG. 5A shows an alternate embodiment of substrates 30 of flex circuit electrode assembly 84 that incorporates radiopaque markers (e.g., 602, 604, 606), which will be described in greater detail below. In FIG. 5A, electrodes 33 and other details shown in FIG. 5A have been hidden for clarity.

With reference to FIGS. 3, 5, and 5A, each substrate 30 has a proximal tail 31P and a distal tail 31D. The proximal tail 31P is tucked under and fastened to the catheter 24 by a proximal ring 28P mounted on the proximal shaft portion 82 of the shaft 70. The distal tail 31D is tucked under and fastened to the catheter 24 by a distal ring (not shown). Either or both sets of tails 31D and 31P may be further covered by a respective semispherical cap, such as distal cap 28D. One or more electrodes 33 on each substrate come into galvanic contract with the ostium 11 during an ablation procedure, during which electrical current flows from the electrodes 33 to the ostium 11, as shown in FIG. 4.

For simplicity, the flex circuit electrode assembly 84 is described with respect to one of its substrates 30 as shown in FIG. 6, although it is understood that following description may apply to each substrate of the assembly. The flex circuit electrode assembly 84 includes a flexible and resilient sheet substrate 34, constructed of suitable bio-compatible materials, for example, polyimide. In some embodiments, the sheet substrate 34 has a greater heat resistance (or a higher melting temperature) compared to that of the balloon membrane 26. In some embodiments, the substrate 34 is constructed of a thermoset material having a decomposition temperature that is higher than the melting temperature of the balloon membrane 26 by approximately 100 degrees Celsius or more.

The substrate 34 is formed with one or more irrigation pores or apertures 35 that are in alignment with the irrigation apertures 27 of the balloon member 26 so that fluid passing through the irrigation apertures 27 and 35 can pass to the ablation site on the ostium.

The substrate 34 has a first or outer surface 36 facing away from the balloon membrane 26, and a second or inner surface 37 facing the balloon membrane 26. On its outer surface 36, the substrate 34 supports and carries the contact electrodes 33 adapted for tissue contact with the ostium. On its inner surface 37, the substrate 34 supports and carries a wiring electrode 38. The contact electrode 33 delivers RF energy to the ostium during ablation or is connected to a thermocouple junction for temperature sensing of the ostium. In the illustrated embodiment, the contact electrode 33 has a longitudinally elongated portion 40 and a plurality of thin transversal linear portions or fingers 41 extending generally perpendicularly from each lateral side of the elongated portion 40 between enlarged proximal and distal ends 42P and 42D, generally evenly spaced therebetween. The elongated portion 40 has a greater width and each of the fingers has a generally uniform lesser width. Accordingly, the configuration or trace of the contact electrode 33 may resemble a "fishbone" but it should be noted that the invention is not limited to such configuration. In contrast to an area or "patch" ablation electrode, the fingers 41 of the contact electrode 33 advantageously increase the circumferential or equatorial contact surface of the contact electrode 33 with the ostium while void regions 43 between adjacent fingers 41 advantageously allow the balloon 80 to collapse inwardly or expand radially as needed at locations along its equator. In the illustrated embodiment, the fingers 41 have different lengths, some being longer, others being shorter. For example, the plurality of fingers includes a distal finger, a proximal finger and fingers therebetween, where each of the fingers in between has a shorter adjacent finger. For example, each finger has a length different from its distal or proximal immediately adjacent neighboring finger(s) such that the length of each finger generally follows the tapered configuration of each substrate 30. In the illustrated embodiment, there are 22 fingers extending across (past each lateral side of) the elongated portion 40, with the longest finger being the third finger from the enlarged proximal end 42P. In some embodiments, the contact electrode 33 includes gold 58B with a seed layer between the gold 58B and the membrane 26. The seed layer may include titanium, tungsten, palladium, silver, or combinations thereof.

Formed within the contact electrode 33 are one or more exclusion zones 47, each surrounding an irrigation aperture 35 formed in the substrate 34. The exclusion zones 47 are voids purposefully formed in the contact electrode 33, as explained in detail further below, so as to avoid damage to the contact electrode 33 during construction of the electrode assembly 84 in accommodating the irrigation apertures 35 at their locations and in their function.

Also formed in the contact electrode 33 are one or more conductive blind vias 48 which are conductive or metallic formations that extend through through-holes in the substrate 34 and are configured as electrical conduits connecting the contact electrode 33 on the outer surface 36 and the wiring electrode 38 on the inner surface 37. It is understood that "conductive" is used herein interchangeably with "metallic" in all relevant instances.

In the illustrated embodiment, the contact electrode 33 measures longitudinally between about 0.1 inch and 1.0 inch, and preferably between about 0.5 inch and 0.7 inch, and more preferably about 0.57 inch, and has four exclusion zones 47 and nine blind vias 48.

On the inner surface 37 of the substrate 34, the wiring electrode 38 is generally configured as an elongated body generally similar in shape and size to the elongated portion 40 of the contact electrode 33. The wiring electrode 38 loosely resembles a "spine" and also functions as a spine in terms of providing a predetermined degree of longitudinal rigidity to each substrate 30 of the electrode assembly 84. The wiring electrode 38 is positioned such that each of the blind vias 48 is in conductive contact with both the contact electrode 33 and the wiring electrode 38. In the illustrated embodiment, the two electrodes 33 and 38 are in longitudinal alignment with other, with all nine blind vias 48 in conductive contact with both electrodes 33 and 38. In some embodiments, the wiring electrode 38 has an inner portion of copper 57 and an outer portion of gold 58.

The wiring electrode 38 is also formed with its exclusion zones 59 around the irrigation apertures 35 in the substrate 34. The wiring electrode 38 is further formed with solder pad portions 61, at least one active 61A, and there may be one or more inactive solder pad portions 61B. The solder pad portions 61A and 61B are extensions from a lateral side of the elongated body of the wiring electrode 38. In the illustrated embodiment, an active solder pad portion 61A is formed at about a mid-location along the elongated body, and a respective inactive solder pad portion 61B is provided at each of the enlarged distal end 42D and the enlarged proximal end 42P.

Attached, e.g., by a solder weld 63, to the active solder pad portion 61A are the wire pair, e.g., a constantan wire 51 and a copper wire 53. The copper wire 53 provides a lead wire to the wiring electrode 33, and the copper wire 53 and the constantan wire 51 provide a thermocouple whose junction is at solder weld 63. The wire pair 51/53 are passed through a through-hole 29 formed in the membrane 26. It is understood that, in other embodiments in the absence of the through-hole 29, the wire pair 51/53 may run between the membrane 26 and the substrate 34 and further proximally between the membrane 26 and the proximal tail 31P until the wire pair 51/53 enters the tubular shaft 70 via another through-hole (not shown) formed in the tubular shaft sidewall closer to the proximal ring 28.

The flex circuit electrode assembly 84, including the substrates 30 and the tails 31P and 31D, is affixed to the balloon membrane 26 such that the outer surface 36 of the substrate 34 is exposed and the inner surface 37 of the substrate 34 is affixed to the balloon membrane 26, with the wiring electrode 38 and wire pair 51/53 sandwiched between the substrate 34 and the balloon membrane 26. The irrigation apertures 35 in the substrate 34 are aligned with the irrigation apertures 27 in the balloon membrane 26. The exclusion zones 59 in the wiring electrode 38 and the exclusion zones 47 in the contact electrode 33 are concentrically aligned with each other, as well as with the irrigation apertures 27 and 35 in balloon 26 and substrate 34, respectively.

Methods of constructing a diagnostic/therapeutic catheter in accordance with the foregoing disclosure may be found in U.S. Patent Application No. 15/360,966, published as U.S. Patent Application Publication No. 2017/0312022.

### Radiopaque Markers

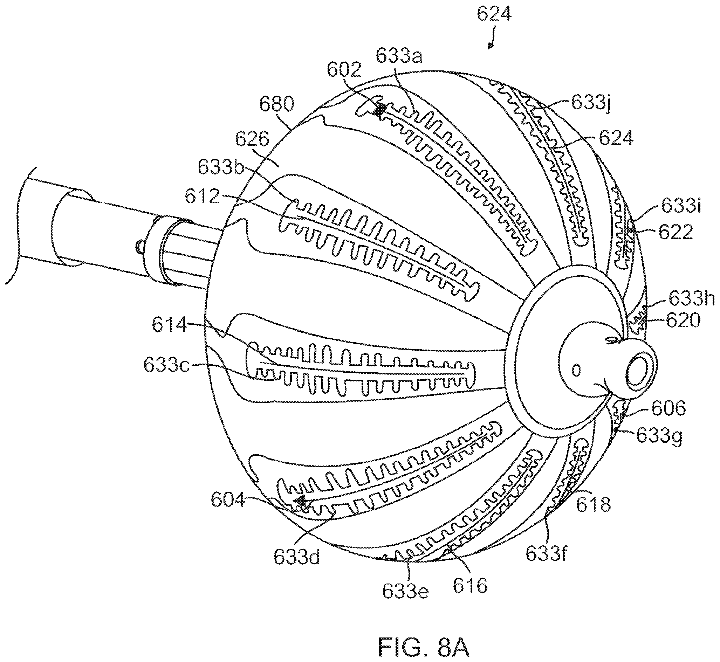

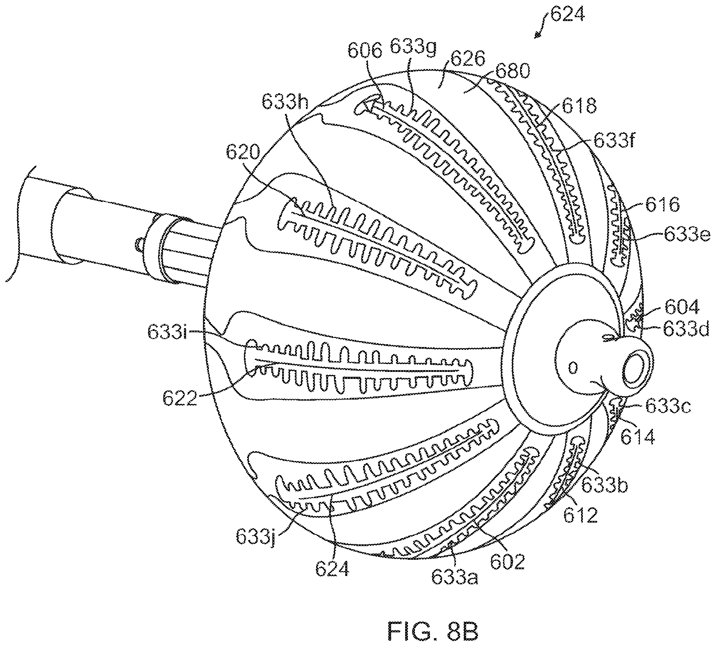

Radiopaque markers made from materials that are opaque to x-rays may be incorporated into diagnostic/therapeutic catheter 24 to assist in visualizing diagnostic/therapeutic catheter 24 and the position of its electrodes 33 during a fluoroscopic procedure. Fluoroscopic images are two-dimensional whereas portions of diagnostic/therapeutic catheter 24 and the cardiac anatomy that diagnostic/therapeutic catheter 24 is intended to ablate with electrodes 33, are three-dimensional objects. Accordingly, fluoroscopic images reflecting diagnostic/therapeutic catheter 24 deployed within the heart may be visually ambiguous, i.e., bi-stable, such that a user (e.g., surgeon) may have difficulty differentiating between the electrodes 33 and how each individual electrode 33 contacts cardiac tissue. Radiopaque markers (e.g., 602, 604, 606 of FIGS. 5A, 8A, and 8B) may assist a user's ability to differentiate between the various contact electrodes 33 and how they contact tissue. FIG. 5B is a schematic representation of how balloon 80 of diagnostic/therapeutic catheter 24, outfitted with the substrates 30 of FIG. 5A (which incorporate radiopaque markers, e.g., 602,604, and 606) may appear in a fluoroscopic image captured when balloon 80 has been deployed and expanded within a subject.

In various embodiments, radiopaque markers may be incorporated onto at least some of the various electrodes 33 of flex-circuit electrode assembly 84 disposed upon balloon 80 such that, during a fluoroscopic procedure, a user may readily identify which electrode 33 is which. Although electrodes 33 may be metallic, e.g., made from gold, and thus radiopaque, additional radiopaque material may be provided thereon as the marker, to darken its fluoroscopic representation. Suitable materials include, e.g., tungsten, gold, bismuth, and barium sulfate. Alternatively, or additionally, the shape of certain individual electrodes 33 may differ somewhat from the others to assist a user in identifying that electrode.

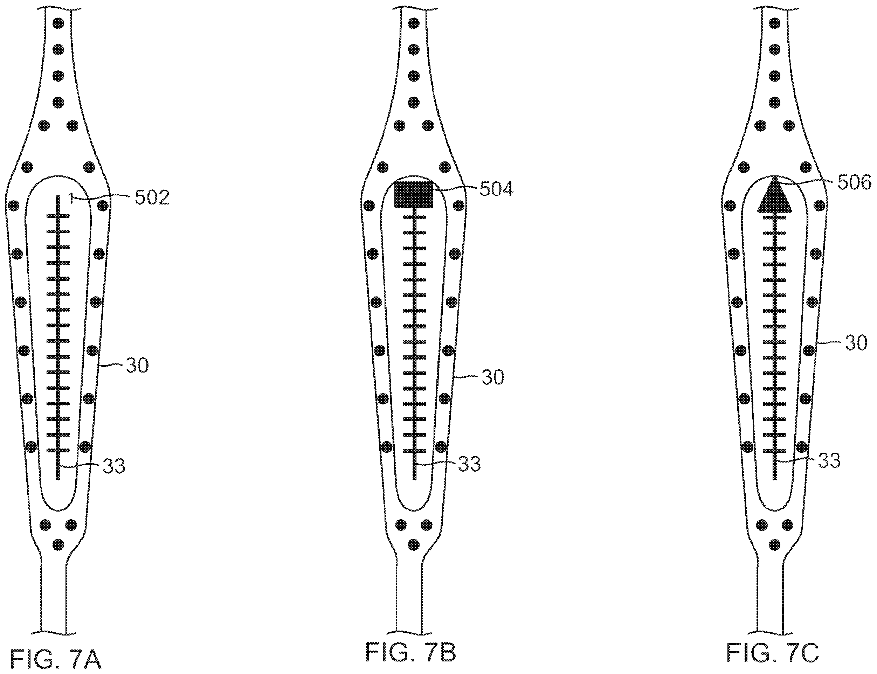

In the exemplary embodiments discussed above in relation to FIGS. 5A and 5B, ten electrodes 33 are provided, one on each substrate 30, each including a radiopaque marker. The ten electrodes 33 can be disposed in any configuration about the longitudinal axis 78. In the preferred embodiments, the electrodes 33 are equiangularly disposed about the longitudinal axis 78 (FIG. 3). Further examples of markers are included in FIGS. 7A-7K, each of which shows a close-up view of a portion of one substrate 30 from FIG. 5. FIGS. 7A- 7K have been modified relative to FIG. 5 to show radiopaque markers having different forms disposed on or incorporated into substrate 30 or electrode 33. Any of the substrates 30 or electrodes 33 shown in these figures may be substituted for any of the unmarked versions shown in FIGS. 2-5, and 6 to design diagnostic/therapeutic catheter 24 such that at least one, but preferably at least two individual substrates 30, electrodes 33, or flex-circuit assemblies 84 may be distinguished from the others under fluoroscopy.

FIG. 7A shows a substrate 30 having a marker 502 in the form of an alphanumeric symbol, e.g., a number or a letter. As shown the numeral is "1," but it should be understood that the form may be any alphanumeric symbol that makes sense to include given the number of substrates 30 or electrodes 33 on balloon 80. For example, where 10 electrodes 33 are used, a first substrate may have a marker 502 in the form of a "1," another substrate may have a marker in the form of a "2", another substrate may have a marker in the form of a "3", another substrate may have a marker in the form of a "4", another substrate may have a marker in the form of a "5", another substrate may have a marker in the form of a "6", another substrate may have a marker in the form of a "7", another substrate may have a marker in the form of a "8", another substrate may have a marker in the form of a "9", and another or ultimate substrate may have a marker in the form of a "10" or an "X".

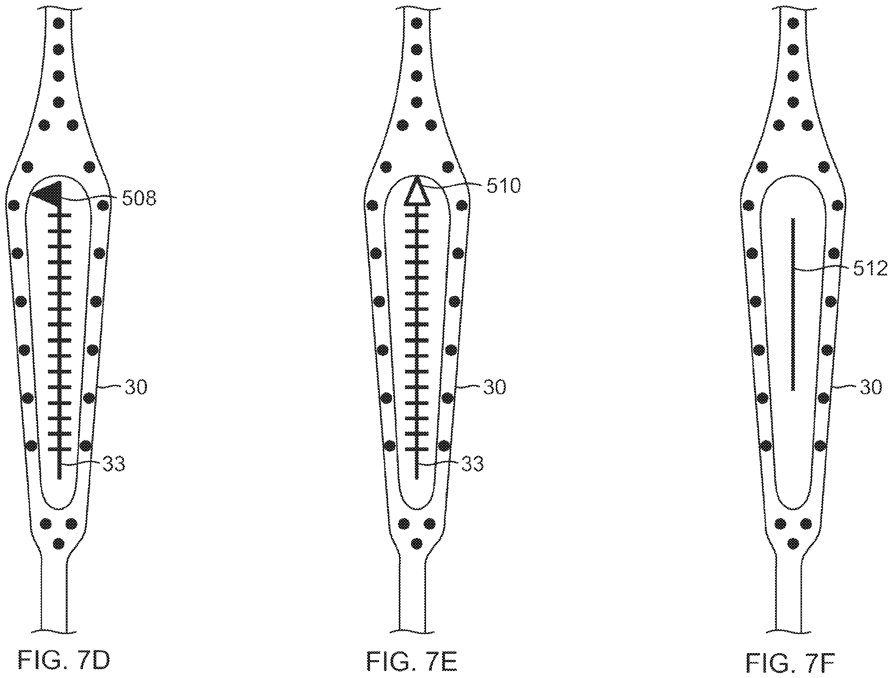

A substrate 30 may also have a marker in the form of a polygon. For example: as shown in FIG. 7B, marker 504 is a rectangle; as shown in FIG. 7C, marker 506 is a triangle or arrowhead pointing along substrate 30; and as shown in FIG. 7D, marker 508 is a triangle or arrowhead pointing perpendicular to substrate 30. As seen in FIG. 7D, marker 508 is provided in a form whereby it is asymmetric about a centerline passing through substrate 30, or, when the flex-circuit assembly 84 is mounted upon balloon 80, is asymmetric about a centerline that is parallel to the longitudinal axis of the balloon. As shown in FIGS. 7B-7D, the polygonal markers 504, 506, and 508 are shown opaque. However, they may also be provided hollow. For example, as shown in FIG. 7E, marker 510 has the form of a hollow polygon in the form of a triangle or arrowhead pointing along substrate 30.

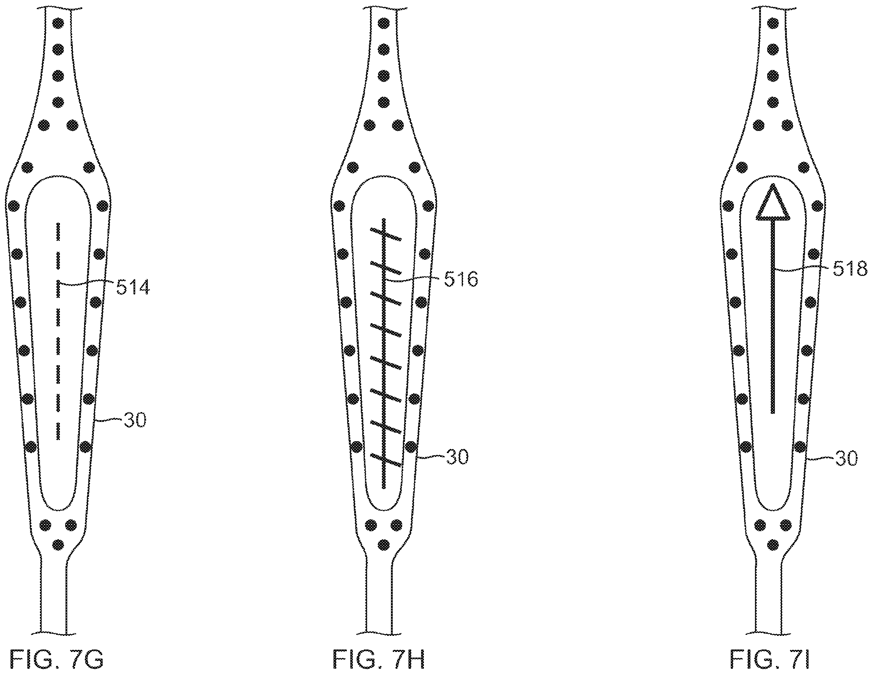

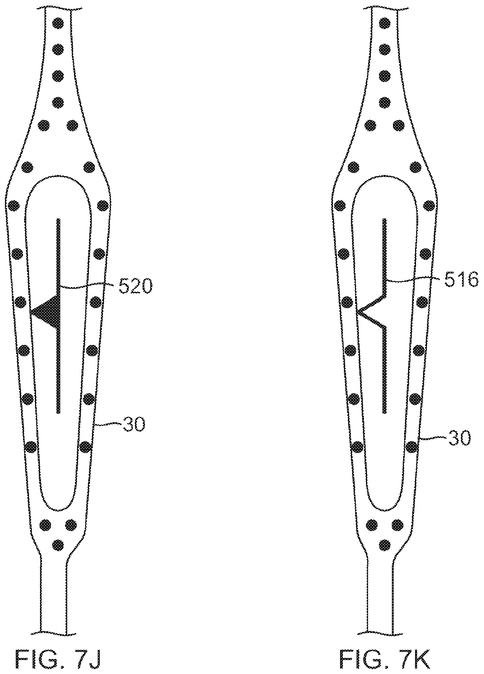

A marker may also have the form of a line or spine that extends along a substrate 30, either disposed thereon or incorporated therein. For example, marker 512 may have the form of a solid line (FIG. 7F), marker 514 may have the form of a dashed line (FIG. 7G), or marker 516 may have the form of a hashed line (FIG. 7H). The markers may also have the form of a line or spine combined with other forms, e.g., alphanumeric symbols or polygons. For example, as shown in FIG. 7I, marker 518 has the form of a solid line connected to a hollow triangle or arrowhead at an end of substrate 30 or electrode 33. As shown, the triangle of marker 518 may be considered at the proximal end of the line, because, referring to FIG. 2 and how substrate 30 is secured to balloon 80, the triangle would be on the end of electrode 33 that is furthest from distal shaft end 88. However, a marker may also include a line or spine with a polygonal form disposed at a location between the two ends of the line or spine. For example, as shown in FIG. 7J, marker 520 includes a line with a triangle or arrowhead disposed proximate to the midpoint of the line and pointing perpendicular to the line. In a variation reflected in FIG. 7K, marker 522 is similar to marker 520, but with the portion of the line that overlaps the base of the triangle removed. Hence, as used herein the, term "form" indicates suitable indicia on the electrode to allow a user to distinguish the orientation of the catheter balloon by a suitable imaging device (e.g., fluoroscopic or MRI). For purposes of demonstrating the markers in FIGS. 7F-7K, features certain features visible in FIG. 5 (e.g., electrodes 33) are hidden in FIGS. 7F-7K.

The markers described above in connection with FIGS. 7A - 7K may be incorporated onto at least one of the individual substrates 30 or electrodes 33 such that any substrate 30 or electrode 33 having a marker disposed thereon may be readily distinguished from those that do not, including when diagnostic/therapeutic catheter 24 is observed under fluoroscopy during an ablation procedure. In some embodiments, three substrates 30 or electrodes 33 include markers. In some embodiments, all of the substrates 30 or electrodes 33 include markers.

FIGS. 8A and 8B show two views of a distal end of a diagnostic/therapeutic catheter that includes a balloon 680 having a membrane 626, as detailed above concerning balloon 80 and membrane 26. In FIG. 8B, balloon 680 is rotated approximately 160 degrees relative to how it is shown in FIG. 8A. Balloon 680 may have a distal end and a proximal end defining a longitudinal axis. Flex-circuit assemblies, akin to flex-circuit assembly 84, may be assembled onto the balloon. Each assembly may include an electrode, akin to electrode 33. For example, in the case where ten assemblies are included, catheter 624 is provided with 10 electrodes 633a-633j disposed on balloon 680. One or more of these electrodes 633a-633j may incorporate radiopaque markers. For example, a first electrode 633a may include a first radiopaque marker 602 of a first form and a second electrode 633d may include a second radiopaque marker 604 of a second form that is different from the first form of first marker 602. For example, first radiopaque marker 602 may have the form of a solid line connected to a solid rectangle and second radiopaque marker 604 may have the form of a solid line connected to a solid triangle. A third electrode 633g may also include a radiopaque marker 606 having a form that is different from the first form of first marker 602 and the second form of second marker 604. For example, third radiopaque marker 606 may have the form of a solid line connected to a hollow triangle. FIGS. 8A and 8B should be considered exemplary. The markers may have alternate forms, such as those provided in FIGS. 7A-7K. Therefore, in certain embodiments, marker 602, 604, or 606 may have a polygonal form that is, e.g., rectangular or triangular. Further, in certain embodiments, one or more may be hollow, and one or more may be solid. One or more of these markers include a solid line, a dotted line, or a hashed line. Further, as shown in FIGS. 8A and 8B, the other electrodes 633b, 633c, 633e, 633f, 633h, 633i, and 633j may include markers 612, 614, 616, 618, 620, 622, and 624, respectively, each having the form of a solid line. In certain embodiments, one or more of these solid lines may be replaced by a dashed line or a hashed line.

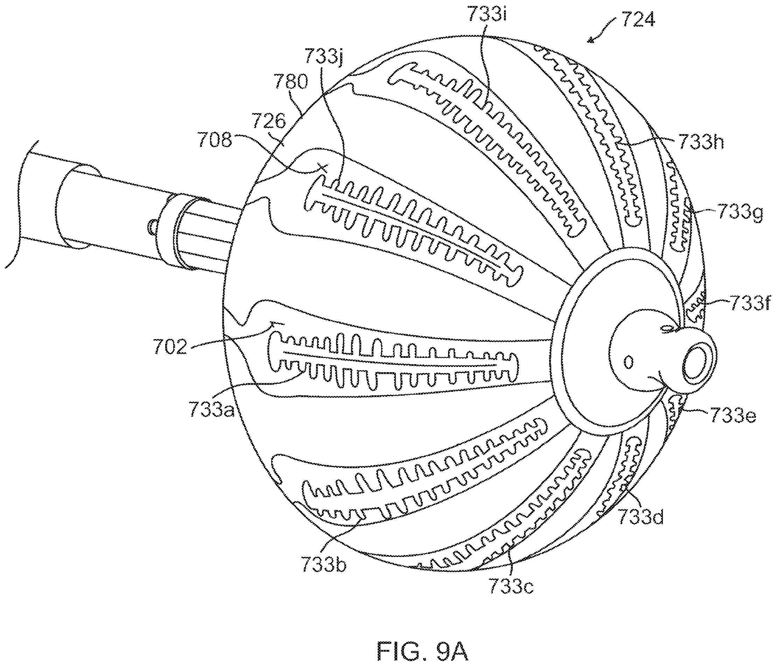

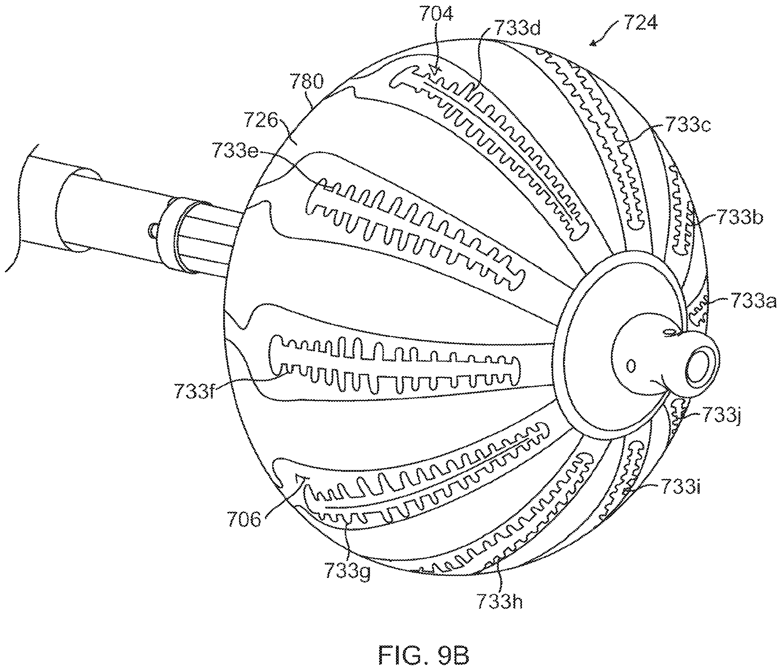

Alternatively, as shown in FIGS. 9A and 9B, the markers may take the form of alphanumeric symbols. In FIG. 9B, balloon 780 is rotated approximately 160 degrees relative to how it is shown in FIG. 9A. In some embodiments, first radiopaque marker 702 may have the form of a "1" and second radiopaque marker 704 may have the form of a numeral greater than "1" corresponding to the electrode's counted designation or position relative to the first electrode, e.g., the "fourth electrode." As shown, the second electrode 733d may be considered the "fourth electrode" because there are two electrodes between electrode 733a (i.e., the "first electrode") and electrode 733d. Thus, in this example, second marker 704 has the form of a "4." Along these lines, additional electrodes may also include markers having forms of numerals. For example, in the instance where the third electrode's counted designation relative to the first electrode is the "seventh electrode," the third electrode is electrode 733g. Thus, in this example, third electrode 733g may include a third marker 706 having the form of a "7." In alternative embodiments at least one of the remaining electrodes may also include a marker having an alphanumeric form. For example, the ultimate electrode, i.e., the electrode adjacent to the first electrode that is not the "second electrode," e.g., electrode 733j, may include a marker 708 having the form of a "10" or an "X."

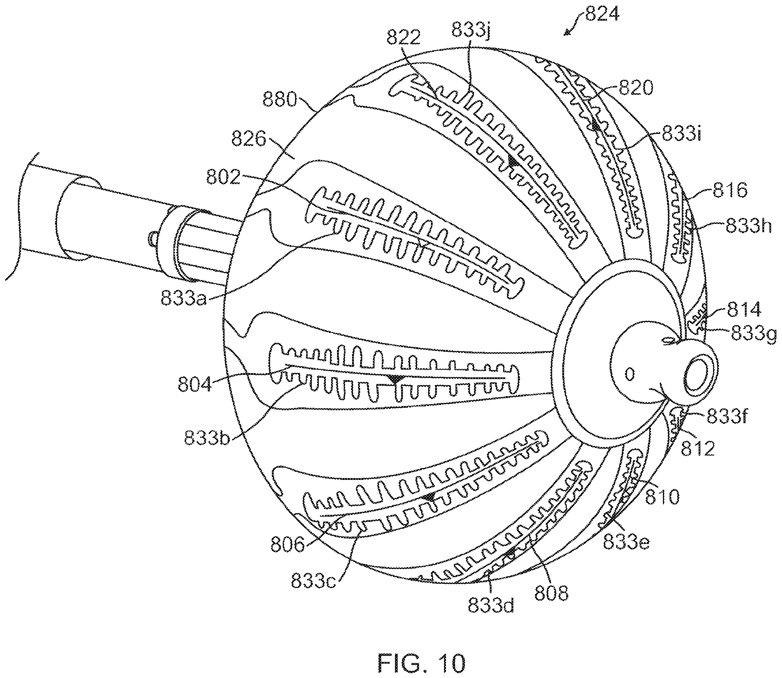

Alternatively, markers may be provided on the electrodes that include an asymmetry relative to each electrode or the balloon, which asymmetry may be used to provide further indications of the three-dimensionality of the balloon and the positions of the electrodes thereon in a bi-stable fluoroscopic image. For example, referring to FIG. 10, ten electrodes 833a-833j are disposed on balloon 880. Each electrode 833a-833j may include a radiopaque marker (e.g., 802, 804, 806, 808, 810, etc.) having the form of an arrowhead or triangle that points to an adjacent electrode viewed clockwise or counterclockwise to the balloon. As shown in FIG. 10, the arrows point to the adjacent electrode in accordance with the so-called "right-hand rule." That is, a user's thumb of her right hand is directed along the catheter's shaft toward the distal end of the catheter shaft such that the user's other fingers on her right-hand curl around the balloon in the direction the arrows point. Alternatively, a "left-hand rule" may be applied. For example, referring to FIG. 5B, which is a schematic of a fluoroscopic image of balloon 80, a user's left thumb may point toward the distal end of the catheter such that her fingers curl around balloon 80 from marker 602, over marker 604, and then over marker 606. A person of skill in the art will appreciate that other conventions for relating the relative positions of the markers and the electrodes exist. The specific convention for relating the relative positions of each markers to the other is not critical in and of itself. What is important is that the user knows the convention and can use it to relate the relative positions of each marker and electrode to the others.

To further distinguish electrode 833a from the others, one or more of the markers may be modified or changed relative to the other markers. For example, marker 802 on electrode 833a may be a hollow arrow while the other markers may be solid arrows. Alternatively, marker 802 may include a double arrow while the makers may include only single arrows.

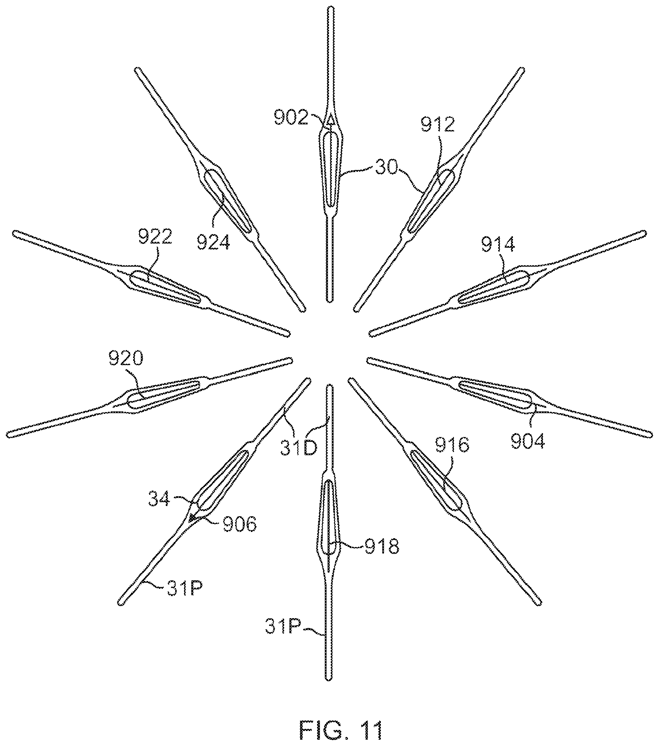

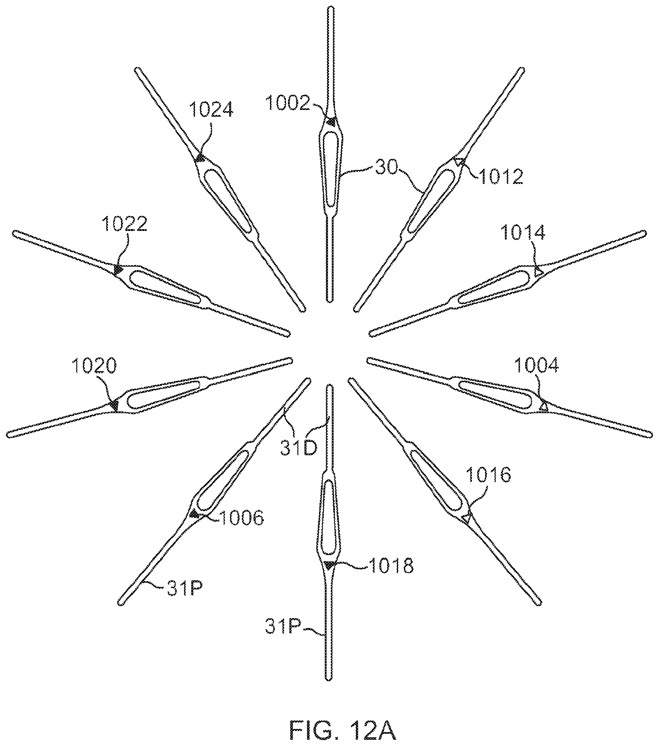

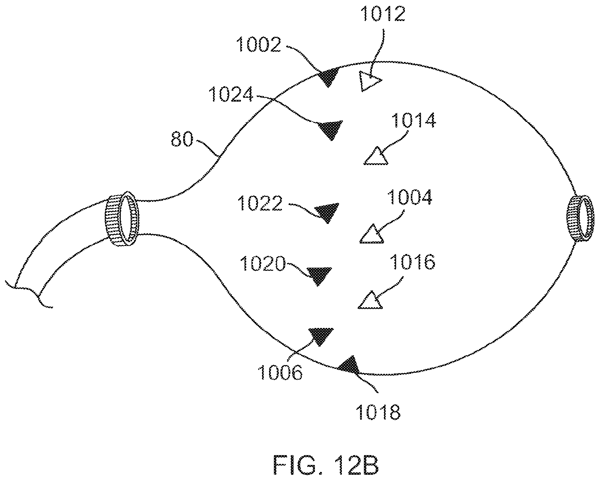

Additional embodiments of a flex-circuit assembly that includes substrates 30 and that may be incorporated onto a balloon, e.g., balloon 80 of diagnostic/therapeutic catheter 24, are shown in FIGS. 11-14. In FIG. 11, at least one marker 902 includes a hollow triangle atop a spine and at least one other marker 906 includes a solid triangle atop a spine. In FIG. 12A, six adjacent markers (1002, 1024, 1022, 1020, 1006, and 1018) each include a solid arrowhead and the remaining four adjacent markers (1016, 1004, 1014, and 1012) each include a hollow arrowhead. Each of the arrowheads, whether solid or hollow, are oriented to point to the adjacent marker (e.g., arrow head of marker 1018 points to marker 1016). FIG. 12B reflects how balloon 80, outfitted with the flex-circuit assembly of FIG. 12A, may appear when viewed fluoroscopically. Each of the arrowheads may provide a user viewing the image with an indication of which marker is which, where each marker is on the surface of balloon 80, and where each marker is with respect to anatomy of a subject. As will be explained in greater detail below, in particular, a user may determine which marker is closest to a subject's esophagus within the subject's left atrium.

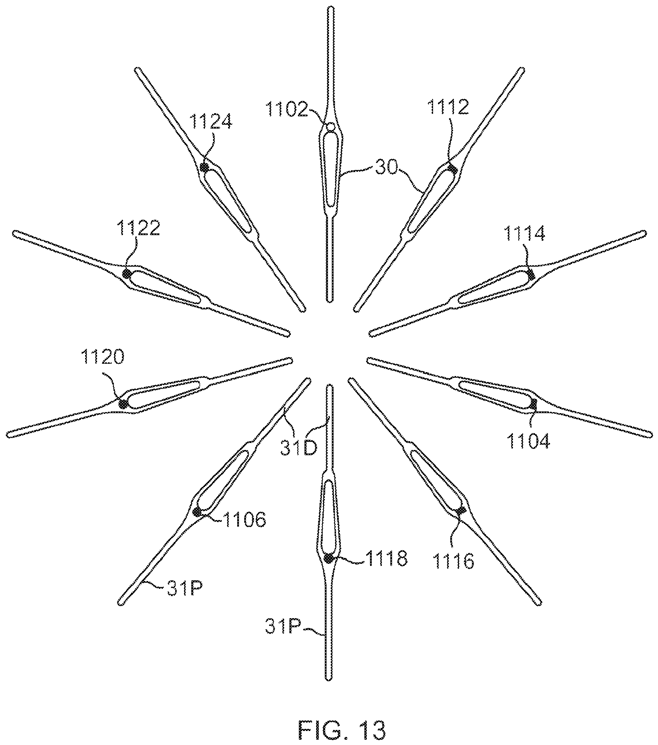

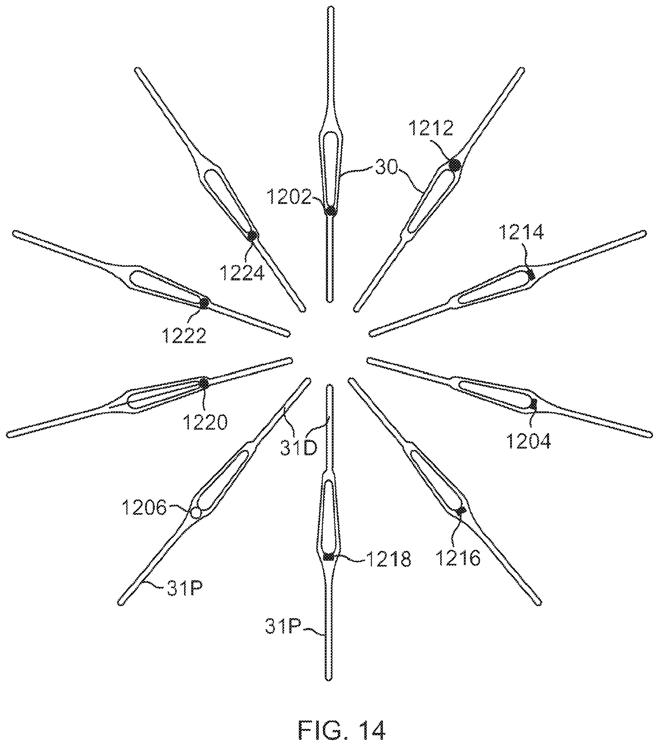

In FIG. 13, some markers may include a hollow circle, e.g., marker 1102, or a solid circle, e.g., marker 1124. Other markers may include a solid rectangle, e.g., marker 1112. FIG. 14 shows that the size and position of the markers may be varied. For example, marker 1212 includes a circle that is larger than the circle of, e.g., marker 1202. Further marker 1212 is disposed at one end of substrate 30, while marker 1202 is disposed at the other end.

In operation, any embodiments incorporating radiopaque markers may facilitate a user's ability to determine the position or orientation of a balloon (e.g., balloon 680 of FIGS. 8A and 8B) within an anatomical structure, e.g., a heart, of a subject because the radiopaque markers are readily visible in a fluoroscopic image. This increased visibility may assist a user's ability to achieve desired results (i.e., proper ablation) without causing unnecessary trauma (e.g., an unwanted lesion) to the subject's anatomy, such as the subject's esophagus. For example, successful ablation of tissue with radio-frequency energy from an electrode (e.g., 633a-633j in FIGS. 8A and 8B) typically requires substantial contact between the electrode and the tissue, often a precise piece of tissue. However, it is undesirable to ablate non-diseased portions of the heart, e.g., away from the pulmonary vein ostium, or the precise piece of tissue. Furthermore, if an incorrect electrode is activated, or is provided with an incorrect energization, other anatomical structures, e.g., a portion of an esophagus close to the left atrium, may be injured. Therefore, with the radiopaque markers, the user can visualize which electrodes are in proper contact before activating an electrode. That is, a user may avoid activating an electrode when it is not in an appropriate position for ablating tissue (e.g., when the electrode does not contact tissue). By providing the user with a visual indication of each marker, and thus, electrode, a user may selectively activate and deactivate individual electrodes on the balloon. Furthermore, a user can tailor the amount of energy or energization each electrode receives based on its location within the heart. Again, by way of example, a user may position a balloon against a pulmonary vein ostium. Viewing the fluoroscopic image, she may see that, e.g., markers 606 and 618 are in contact with tissue of the ostium, but that the remaining markers are not. Thus, she may determine that the corresponding electrodes (e.g., 633f and 633g in FIG. 8B) are in contact with tissue such that ablation of tissue may be achieved by activating these two electrodes. Concomitantly, she may also determine that the remaining electrodes should not be activated. However, at this point, she still needs to relate which switches, e.g., within ablation module 54, activate electrodes 633f and 633g such that only these electrodes, and not the others, will receive radiofrequency energy from the radiofrequency generator. To do so, the user may be trained to know a correspondence between electrodes and markers. For example, she may understand that: 1) first marker 602 (which as shown in e.g., FIG. 5A includes a solid rectangle), corresponds to first electrode 633a; 2) second marker 604 (which as shown in e.g., FIG. 5A includes a solid triangle) corresponds to second electrode 633d; and 3) third marker 606 corresponds to third electrode 633g. She may also understand that there are two electrodes (i.e., 633b and 633c) between electrodes 633a and 633d. She may also understand that there are two electrodes (i.e., 633e and 633f) between electrodes 633d and 633g. Accordingly, in standard spoken English, she may understand electrode 633a as "the first electrode," electrode 633d as "the fourth electrode," and electrode 633g as "the seventh electrode." Finally, she may also understand that the markers are disposed circumferentially about the balloon in a clockwise or counterclockwise fashion, or preferably, according to the so-called "right-hand rule" or "left-hand rule" described above.

Thus, upon observing that the hollow-arrow of marker 606 contacts tissue, she may quickly deduce that electrode 633g (in her mind, "the seventh electrode") is in proper contact with tissue and may be activated with a first switch (in her mind, likely a "seventh switch") of ablation module 54. Further, applying the right-hand rule, she may then deduce that electrode 633f (in her mind, "the sixth electrode") may be activated with a second switch (in her mind, likely a "sixth switch") of ablation module 54. Accordingly, she may activate electrodes 633f and 633g with increased confidence that she will achieve a successful ablation procedure and outcome for her subject.

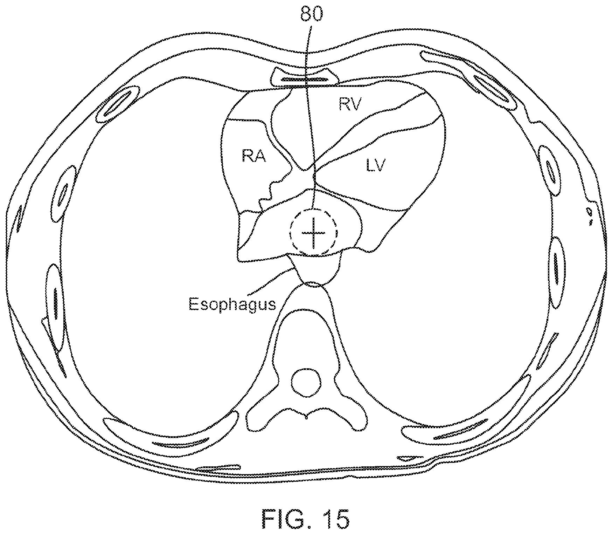

Furthermore, the user may observe that balloon 80 is disposed proximate to a portion of the left atrium that is proximate to another anatomical structure of the subject, such as the subject's esophagus, as seen in FIG. 15, in which balloon 80 is indicated as a dotted-line circle. To avoid causing unwanted trauma, such as a lesion, to the esophagus, the user may tailor or adjust the amount of energy the electrodes receive when they are proximate to the esophagus. That is, the user may energize the electrode or electrodes (indexed or referenced to marker 902) closest to the esophagus at a first energization setting that is lower than a second energization setting used when those same or other electrodes (indexed or referenced to marker 906) may be disposed at other locations away from the esophagus. As used herein, the term "energization setting" indicates information concerning energy received by an electrode from ablation module 54. For example, a constant amount of power (with units of e.g., watts) may be delivered over a constant span of time. The constant amount of power may be predetermined. The constant amount of time may be predetermined. Alternatively, the power may be varied (e.g., by magnitude, frequency, as a function of time, or as a function of impedance, which itself may be function of, e.g., lesion size). Alternatively, the amount of time may be varied (e.g., as a function of impedance). The phase of the delivered power may be in-phase or out-of-phase with the power delivered to other electrodes. In the exemplary embodiments, the power provided to ablate tissue is dependent on the measured impedance and can be from about 10 watts to about 250 watts. The duration of the energy application can be from about 4 seconds to about 90 seconds in order to form an effective lesion.

Viewed together, diagnostic/therapeutic catheter 24, which includes a balloon (e.g., balloon 80), operating console 15, which includes ablation module 54, and wires connecting electrodes disposed on substrates of the balloon to outputs of ablation module 54, may be considered components an electrophysiology system for providing ablative therapy to a patient. Ablation module 54 may include at least one radio-frequency generator. For example, it may include the same number of radio-frequency generators as the balloon includes electrodes. In this manner, the frequency of the energy delivered to each electrode may be tailored relative to the other electrodes, e.g., based on changing impedance, which is an indicator of tissue ablation. Each wire may be a portion of an electrical path between a respective electrode and ablation-module output or radio-frequency generator. At least a portion of each wire may be disposed within the diagnostic/therapeutic catheter. Alternatively, an electrical path may consist of a single wire that directly connects one electrode to a respective ablation-module output. Switches may be used to selectively activate and deactivate each electrode, i.e., to connect and disconnect each electrode from the generator. In some embodiments, each switch may be included in each electrical path. In some embodiments each switch may be included within ablation module 54, e.g., between the signal generator and the output, or between a power source and the generator. Each electrical path may further include an additional wire or electrical connector, e.g., a pin connector, which may be used to facilitate connecting the catheter to operating module 15, which may include a direct connection or an indirect connection to ablation module 54. Each switch may be manually controlled, e.g., by a user-inter interface mechanism, such as controls 49 of FIG. 1. In some embodiments, mechanical switches may be provided within the electrical paths outside of operating module 15. As detailed above, the substrates or electrodes of the balloon of this system may include radiopaque markers, which a user may utilize to determine from a two dimensional or bi-stable image, e.g., a fluoroscopic image, relative locations of the markers with respect to each other, to determine which electrode(s) to activate, and to determine the correct switch for activating that or those electrode(s).

By virtue of the embodiments illustrated and described herein, applicant has devised a method of ablating tissues selectively along a tissue surface, e.g., a curved tissue surface, in contact with a balloon of a diagnostic/therapeutic catheter. That is, a user may use the diagnostic/therapeutic catheter described above or the electrophysiology system of which it may be a part, according to various methods and variations to activate at least one electrode while maintaining inactive the other electrodes. For example, the user may provide a diagnostic/therapeutic catheter (e.g., catheter 24) that includes a balloon (e.g., balloon 80) having various substrates (e.g., substrates 30) disposed circumferentially thereon. The substrates may further include radiopaque markers (e.g., markers 602, 604, 606) disposed thereon. In some embodiments, each substrate includes an electrode (e.g., electrode 33) disposed thereon with at least a portion of the marker disposed on the electrode. In some embodiments, ten substrates, each including one electrode and one marker, are employed. The balloon may be positioned at a desired location within a subject's anatomy. For example, the balloon may be positioned within the subject's heart. Specifically, the balloon may be positioned proximate to or within the heart's pulmonary vein ostium. The user may use various visualization techniques known in the art, e.g., fluoroscopy to assist in positioning the balloon at the desired location. Once at the desired location, the user may use that visualization technique to assess the position and orientation of the balloon. Specifically, the user may use the visualization technique to determine the relative positions and orientations of the markers with respect to each other such that the user can further determine the relative positions and orientations of each substrate and each electrode with respect to the others. Furthermore, the user may use the visualization technique to determine whether any of the markers are in contact with the subject's tissue such that the user can further determine whether a corresponding electrode is in contact with the tissue. For example, the user may determine that a first marker, and thus a first electrode contacts tissue at a desired location. Upon that determination, the user may activate the first electrode, e.g., by closing a first switch, e.g., by using controls 49. The user may also determine that a second marker does not contact tissue, or does not contact tissue at a desired location. Upon that determination, the user may determine not to activate the second electrode. Alternatively, the user may determine that the second marker contacts tissue at a desired location. Upon that determination the user may activate the second electrode, e.g., by closing a second switch, e.g., by using controls 49.

In certain variations of the method, the user may determine that the first switch activates the first electrode and that the second switch activates the second electrode. As explained above, it may not be readily apparent to the user which electrode corresponds to which switch, e.g., due to the ambiguous nature of bi-stable images produced fluoroscopically. The markers disposed on the substrates of the balloon may help the user determine which electrode corresponds to which switch. For example, the first electrode may include thereon a first marker having a first form and the second electrode may include thereon a second marker having a second form. The user may correlate the first form, e.g., a rectangle, such as a solid rectangle, to the first switch, and the user may correlate the second form, e.g., a line or triangle, to the second switch. In further variations, including those where the second marker is a line, the user may correlate the second form to the second switch using the right-hand rule or the left-hand rule in association with the first marker.

In one variation of the method, the following steps can be performed by the user to ablate a desired portion of the cardiac tissue: positioning an expandable member proximate to a tissue within a subject's anatomy; viewing an image of the expandable member with a suitable imaging device (e.g., fluoroscopic or x-ray); determining that one or more electrodes adjacent one of the first marker or the second marker contacts the tissue; and energizing the one or more electrodes to ablate the tissue.

Also disclosed herein is a method of ablating a portion of a heart without ablating adjacent anatomical structures or creating a lesion on that other anatomical structures (e.g., esophagus, phrenic nerve or lung) disposed proximate to a left atrium of the heart. In some variations the method is for applying energy to tissue in a left atrium of a subject proximate to an esophagus, phrenic nerve or lung. An expandable member (e.g., balloon 80) may be positioned proximate to the left atrium of the subject's heart. The expandable member may have a longitudinal axis and include a plurality of electrodes (e.g., 30), disposed about the longitudinal axis. For example, between three and fifteen, or ten electrodes may be included. Each electrode may be capable of being energized independently. The method can be achieved by positioning an expandable member proximate to the left atrium; viewing an image of the expandable member as well as the first and second radiopaque markers in the left atrium; determining an orientation of the first and second radiopaque markers with respect to a portion of the left atrium closest to the esophagus, phrenic nerve, or lung, of the subject; moving one of the first and second radiopaque marker to a portion of the left atrium closest to the esophagus, phrenic nerve or lung; and energizing one or more electrodes indexed to the one of the radiopaque markers proximate the portion close to the esophagus, phrenic nerve, or lung, at a lower energization setting as compared to other electrodes. Hence, a transmural lesion may thus be created in the left atrium without injury or substantial injury to adjacent anatomical structures including, for example, esophagus, phrenic nerve or lung.

Any of the examples or embodiments described herein may include various other features in addition to or in lieu of those described above. The teachings, expressions, embodiments, examples, etc. described herein should not be viewed in isolation relative to each other. Various suitable ways in which the teachings herein may be combined should be clear to those skilled in the art in view of the teachings herein.

Having shown and described exemplary embodiments of the subject matter contained herein, further adaptations of the methods and systems described herein may be accomplished by appropriate modifications without departing from the scope of the claims. In addition, where methods and steps described above indicate certain events occurring in certain order, it is intended that certain steps do not have to be performed in the order described but in any order as long as the steps allow the embodiments to function for their intended purposes. Therefore, to the extent there are variations of the invention, which are within the disclosure of the inventions found in the claims, it is the intent that this patent will cover those variations as well. Some such modifications should be apparent to those skilled in the art. For instance, the examples, embodiments, geometries, materials, dimensions, ratios, steps, and the like discussed above are illustrative. Accordingly, the claims should not be limited to the specific details of structure and operation set forth in the written description and drawings.

## Claims

1. An electrophysiology catheter, comprising:
a balloon (80) having a membrane (26), the balloon having a distal end and a proximal end defining a longitudinal axis;
a first substrate (30) disposed on the membrane, the first substrate including a first radiopaque marker (802) of a first form disposed thereon; and
a second substrate (30) disposed on the membrane, the second substrate including a second radiopaque marker (804) of a second form disposed thereon, the second form being different from the first form,
wherein the first form includes a first polygon and the second form includes a second polygon,
wherein the second polygon is asymmetric about a centerline that is parallel to the longitudinal axis of the balloon (80), wherein the second polygon includes an arrowhead, and wherein the second polygon points toward a third substrate (30),
wherein the third substrate includes a third radiopaque marker (806) of a third form disposed thereon, the third form being different from the first form and the second form, and wherein the third form includes a third polygon,
wherein the third polygon is asymmetric about a centerline that is parallel to the longitudinal axis of the balloon (80), wherein the third polygon includes an arrowhead, and wherein the third polygon points toward an ultimate substrate (30).

2. The electrophysiology catheter of claim 1, wherein the first polygon includes a rectangle and the second polygon includes a triangle.

3. The electrophysiology catheter of claim 1, wherein the second polygon is hollow.

4. The electrophysiology catheter of claim 1, wherein the second polygon is solid.

5. The electrophysiology catheter of claim 1, wherein the third polygon includes a triangle.

6. The electrophysiology catheter of claim 1, wherein the third polygon is solid.

7. The electrophysiology catheter of claim 1, wherein the third polygon is hollow.

8. The electrophysiology catheter of claim 1, wherein the first substrate (30), second substrate (30), third substrate (30), and ultimate substrate (30) are disposed circumferentially about the membrane (26) and the ultimate substrate (30) is disposed on the membrane between the third substrate (30) and the first substrate (30).

9. The electrophysiology catheter of claim 8, wherein at least a fourth substrate (30) is disposed between the first substrate (30) and the second substrate (30), and at least a fifth substrate (30) is disposed between the second substrate (30) and the third substrate (30), and optionally wherein the fourth substrate (30) lacks a radiopaque marker and fifth substrate (30) lacks a radiopaque marker.

## Patentansprüche

1. Elektrophysiologischer Katheter, umfassend:
einen Ballon (80) mit einer Membran (26), wobei der Ballon ein distales Ende und ein proximales Ende aufweist, die eine Längsachse definieren;
ein erstes Substrat (30), das auf der Membran angeordnet ist, wobei das erste Substrat einen ersten röntgendichten Marker (802) mit einer darauf angeordneten ersten Form einschließt; und
ein zweites Substrat (30), das auf der Membran angeordnet ist, wobei das zweite Substrat einen zweiten röntgendichten Marker (804) mit einer darauf angeordneten zweiten Form einschließt, wobei die zweite Form sich von der ersten Form unterscheidet,
wobei die erste Form ein erstes Vieleck einschließt und die zweite Form ein zweites Vieleck einschließt,
wobei das zweite Vieleck um eine Mittellinie asymmetrisch ist, die parallel zu der Längsachse des Ballons (80) ist, wobei das zweite Vieleck eine Pfeilspitze einschließt, und wobei das zweite Vieleck in Richtung eines dritten Substrats (30) zeigt,
wobei das dritte Substrat einen röntgendichten Marker (806) mit einer darauf angeordneten dritten Form einschließt, wobei die dritte Form sich von der ersten Form und der zweiten Form unterscheidet, und wobei die dritte Form ein drittes Vieleck einschließt,
wobei das dritte Vieleck um eine Mittellinie asymmetrisch ist, die parallel zu der Längsachse des Ballons (80) ist, wobei das dritte Vieleck eine Pfeilspitze einschließt, und wobei das dritte Vieleck in Richtung eines ultimativen Substrats (30) zeigt.

2. Elektrophysiologischer Katheter nach Anspruch 1, wobei das erste Vieleck ein Rechteck einschließt und das zweite Vieleck ein Dreieck einschließt.

3. Elektrophysiologischer Katheter nach Anspruch 1, wobei das zweite Vieleck hohl ist.

4. Elektrophysiologischer Katheter nach Anspruch 1, wobei das zweite Vieleck ausgefüllt ist.

5. Elektrophysiologischer Katheter nach Anspruch 1, wobei das dritte Vieleck ein Dreieck einschließt.

6. Elektrophysiologischer Katheter nach Anspruch 1, wobei das dritte Vieleck ausgefüllt ist.

7. Elektrophysiologischer Katheter nach Anspruch 1, wobei das dritte Vieleck hohl ist.

8. Elektrophysiologischer Katheter nach Anspruch 1, wobei das erste Substrat (30), das zweite Substrat (30), das dritte Substrat (30) und das ultimative Substrat (30) umlaufend um die Membran (26) angeordnet sind und das ultimative Substrat (30) auf der Membran zwischen dem dritten Substrat (30) und dem ersten Substrat (30) angeordnet ist.

9. Elektrophysiologischer Katheter nach Anspruch 8, wobei mindestens ein viertes Substrat (30) zwischen dem ersten Substrat (30) und dem zweiten Substrat (30) angeordnet ist und mindestens ein fünftes Substrat (30) zwischen dem zweiten Substrat (30) und dem dritten Substrat (30) angeordnet ist, und wobei gegebenenfalls dem vierten Substrat (30) ein röntgendichter Marker fehlt und dem fünften Substrat (30) ein röntgendichter Marker fehlt.

## Revendications

1. Cathéter d'électrophysiologie, comprenant :
un ballonnet (80) ayant une membrane (26), le ballonnet ayant une extrémité distale et une extrémité proximale définissant un axe longitudinal ;
un premier substrat (30) disposé sur la membrane, le premier substrat comprenant un premier marqueur radio-opaque (802) d'une première forme disposé sur celui-ci, et
un deuxième substrat (30) disposé sur la membrane, le deuxième substrat comprenant un deuxième marqueur radio-opaque (804) d'une deuxième forme disposé sur celui-ci, la deuxième forme étant différente de la première forme,
la première forme comprenant un premier polygone et la deuxième forme comprenant un deuxième polygone,
le deuxième polygone étant asymétrique par rapport à une ligne centrale qui est parallèle à l'axe longitudinal du ballonnet (80), le deuxième polygone comprenant une pointe de flèche, et le deuxième polygone pointant vers un troisième substrat (30),
le troisième substrat comprenant un troisième marqueur radio-opaque (806) d'une troisième forme disposé sur celui-ci, la troisième forme étant différente de la première forme et de la deuxième forme, et la troisième forme comprenant un troisième polygone,
le troisième polygone étant asymétrique autour d'une ligne centrale qui est parallèle à l'axe longitudinal du ballonnet (80), le troisième polygone comprenant une pointe de flèche, et le troisième polygone pointant vers un substrat ultime (30).

2. Cathéter d'électrophysiologie selon la revendication 1, le premier polygone comprenant un rectangle et le deuxième polygone comprenant un triangle.

3. Cathéter d'électrophysiologie selon la revendication 1, le deuxième polygone étant creux.

4. Cathéter d'électrophysiologie selon la revendication 1, le deuxième polygone étant plein.

5. Cathéter d'électrophysiologie selon la revendication 1, le troisième polygone comprenant un triangle.

6. Cathéter d'électrophysiologie selon la revendication 1, le troisième polygone étant plein.

7. Cathéter d'électrophysiologie selon la revendication 1, le troisième polygone étant creux.

8. Cathéter d'électrophysiologie selon la revendication 1, le premier substrat (30), le deuxième substrat (30), le troisième substrat (30) et le substrat ultime (30) étant disposés circonférentiellement autour de la membrane (26) et le substrat ultime (30) étant disposé sur la membrane entre le troisième substrat (30) et le premier substrat (30).

9. Cathéter d'électrophysiologie selon la revendication 8, au moins un quatrième substrat (30) étant disposé entre le premier substrat (30) et le deuxième substrat (30), et au moins un cinquième substrat (30) étant disposé entre le deuxième substrat (30) et le troisième substrat (30), et éventuellement le quatrième substrat (30) étant dépourvu de marqueur radio-opaque et le cinquième substrat (30) étant dépourvu de marqueur radio-opaque.
